# EUROPEAN PATENT APPLICATION

(11) **EP 4 760 247 A1**
(43) Date of publication of application: **17.06.2026**
(21) Application number: 25221256.8
(22) Date of filing: 05.12.2025
(51) Int. Cl.: G01N 25/18, G01N 33/00

(54) **SENSOR FOR GAS DETECTION IN AIR**

(30) Priority: 16.12.2024 IT 202400028575
(71) Applicant: STMicroelectronics International N.V., 1228 Plan-les-Ouates, Geneva (CH)
(72) Inventor: CARMINATI, Roberto, 20864 Agrate Brianza (MB) (IT); FERRARI, Giacomo, 20864 Agrate Brianza (MB) (IT); MERLI, Massimiliano, 20864 Agrate Brianza (MB) (IT); NICOLI, Silvia, 20864 Agrate Brianza (MB) (IT)
(74) Representative: Studio Torta S.p.A.

(57) **Abstract**

Sensor (30), of the micro-electromechanical type, for detecting a target gas in air, the sensor (30) comprising: a main body (32) defining a first cavity (34'), buried in the main body (32) and in fluidic communication with an environment external to the sensor (30) in such a way as to allow the flow of air from the external environment towards the first cavity (34'), the main body (32) comprising a first frame portion (42') facing the first cavity (34'); a first suspended mass (38'), suspended in the first cavity (34') and comprising at least one thermally isolated metal-oxide semiconductor (TMOS) transistor (20), configured to detect the target gas in air, and at least one heater element (22), configured to heat the first suspended mass (38'); and a first elastic group (40') extending in the first cavity (34') between the first suspended mass (38') and the first frame portion (42'), the first elastic group (40') being configured to elastically couple the first suspended mass (38') to the first frame portion (42').

## Description

### TECHNICAL FIELD

The present invention relates to a sensor for gas detection in air. Furthermore, it relates to a measuring device comprising the sensor, a measuring method performed through the measuring device and a corresponding computer program product.

### BACKGROUND

As is known, different applications require measuring the concentration of specific gases in air. This may be useful, for example, to report the presence of harmful gases in closed environments or to detect the presence of people or the operating state of objects or machinery.

Detecting gases in air is known through thermal conductivity gas sensors.

Thermal conductivity gas sensors are a family of gas sensors based on the measurement of the variation in the thermal conductivity of the air due to the presence of a secondary gas, to be detected, which has a specific and different thermal conductivity.

This type of sensor is particularly effective in the case of hydrogen detection, since hydrogen has a much higher thermal conductivity than other gaseous components commonly present in the air (e.g. nitrogen, oxygen, etc.).

Hydrogen detection is becoming increasingly important for the industry, for example considering the introduction of electric vehicles and fuel cell vehicles on the market. In fact, a possible malfunction of the vehicle may cause a burn-out which releases hydrogen in the air. Since hydrogen is highly flammable, these leakages pose serious safety issues for people, environments and objects surrounding the vehicle.

However, known thermal conductivity sensors are generally bulky, expensive and inaccurate.

For instance, thermal conductivity gas sensors are currently known which incorporate sensitive elements, such as a platinum resistance, placed on a suspended dielectric membrane which is used for both heating and detection. However, in these sensors the power consumption is high due to the limited thermal insulation which may be achieved (in fact, platinum is a metal with quite high thermal conductivity).

Detecting hydrogen accurately, with reduced power consumption and with low-cost solutions, is therefore a widely felt need.

Other types of gas sensors are also known, based on technologies other than thermal conductivity detection.

For instance, an alternative technology is based on gas detection through semiconducting metal oxides (SMOs) which are sensitive to the gas to be detected (in particular, their electrical resistance changes selectively in the presence of such gas). However, these sensors have high power consumption, due to the need to heat the detection resistors to high temperatures, poor reliability, high humidity dependence, and significant drift coming with age, due to the slow degradation of the active material.

For example, document US2024133854A1 relates to a micro-machined sensor and, more in detail, to a fluid sensor for sensing concentration of a fluid or concentration of components of a fluid based on thermal conductivity of the fluid.

Document US2021311006A1 relates generally to micro-electro-mechanical systems (MEMS), such as microhotplate devices, gas sensors including the microhotplate devices, and to related methods of forming and operating the microhotplate devices and gas sensors.

Document US2024272004A1 relates to the field of thermal sensing devices and, more particularly, to a process flow for forming thermally isolated MOS sensors (TMOS).

Document US2024302219A1 relates to the field of thermal sensing devices and, more particularly, to a process flow for forming thermally isolated MOS sensors (TMOS) incorporating mechanical stoppers to increase robustness to mechanical shocks.

Document Zviagintsev et al., "Micro-machined CMOS-SOI transistor (TMOS) thermal sensor operating in air", 2017 IEEE International Conference on Microwaves, Antennas, Communications and Electronic Systems (COMCAS), presents the performance of CMOS-SOI TMOS thermal sensor processed in a well-established 1 µm technology, operating in air.

As a result, none of these known solutions allow an accurate, low-cost, and low-power consumption detection.

### SUMMARY

The aim of the present invention is to provide a sensor, a measuring device, a measuring method and a corresponding computer program product, which overcome the drawbacks of the prior art.

According to the present invention, a sensor, a measuring device, a measuring method and a corresponding computer program product are provided, as defined in the annexed claims which form an integral part of the present description.

### BRIEF DESCRIPTION OF THE DRAWINGS

For a better understanding of the present invention, a preferred embodiment is now described, purely by way of non-limiting example, with reference to the attached drawings, wherein:
- Figure 1 is a block diagram schematically illustrating a measuring device for detecting a target gas in air, according to one embodiment;
- Figure 2 is a cross-sectional view of an assembly comprised in the measuring device of Figure 1, according to one embodiment;
- Figure 3 is a top view with parts hidden of the assembly of Figure 2, according to one embodiment;
- Figure 4 is a top view with parts hidden of a detail of the assembly of Figure 2, according to one embodiment;
- Figure 5 is a top view with parts hidden of a detail of the assembly of Figure 2, according to a different embodiment;
- Figure 6 is a cross-sectional view of the detail of Figure 5 of the assembly of Figure 2, according to one embodiment;
- Figure 7 is a block diagram schematically illustrating a measuring method for detecting a target gas in air, according to one embodiment, performed through the measuring device of Figure 1;
- Figure 8 is a plot showing an example of correlation between physical quantities used in performing the measuring method of Figure 7; and
- Figure 9 is a top view with parts hidden of the assembly of Figure 2, according to a further embodiment.

In particular, the Figures are shown with reference to a triaxial Cartesian system defined by an X-axis, a Y-axis and a Z-axis, orthogonal to each other.

In the following description, elements common to the different embodiments have been indicated with the same reference numbers.

### DETAILED DESCRIPTION OF THE INVENTION

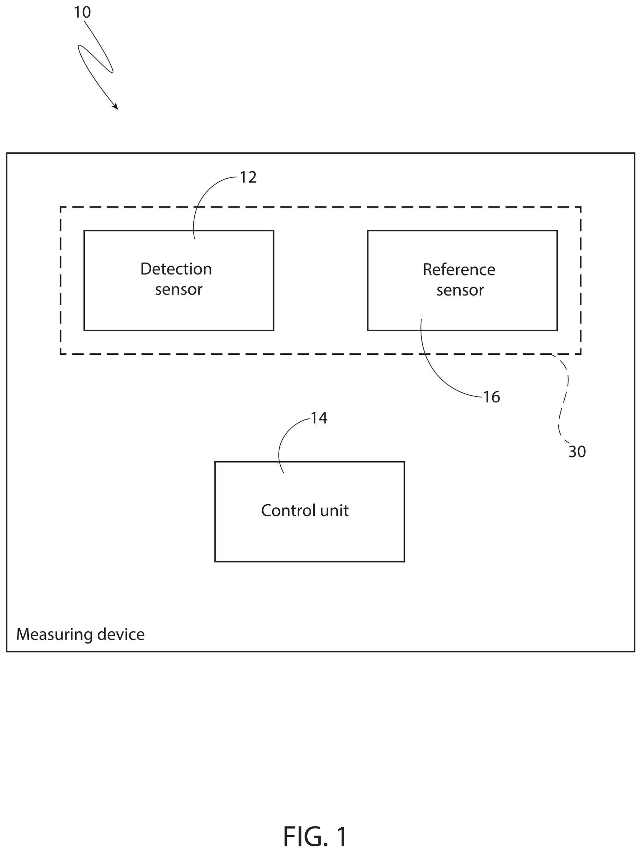

Figure 1 shows a measuring device 10 for detecting a gas (also referred to as target gas or gas to be detected), in particular hydrogen.

The measuring device 10 allows the concentration of the target gas to be detected in the air of the environment in which it is placed.

The measuring device 10 comprises a detection sensor (or first sensor) 12 and a control unit 14, operationally coupled to each other.

The detection sensor 12 is configured to measure the concentration of the target gas in the air and to generate a corresponding detection signal indicative of this concentration measurement.

In particular, the detection signal is an electrical signal, for example of an analogue or digital type, with a value dependent on the concentration of the target gas in the air.

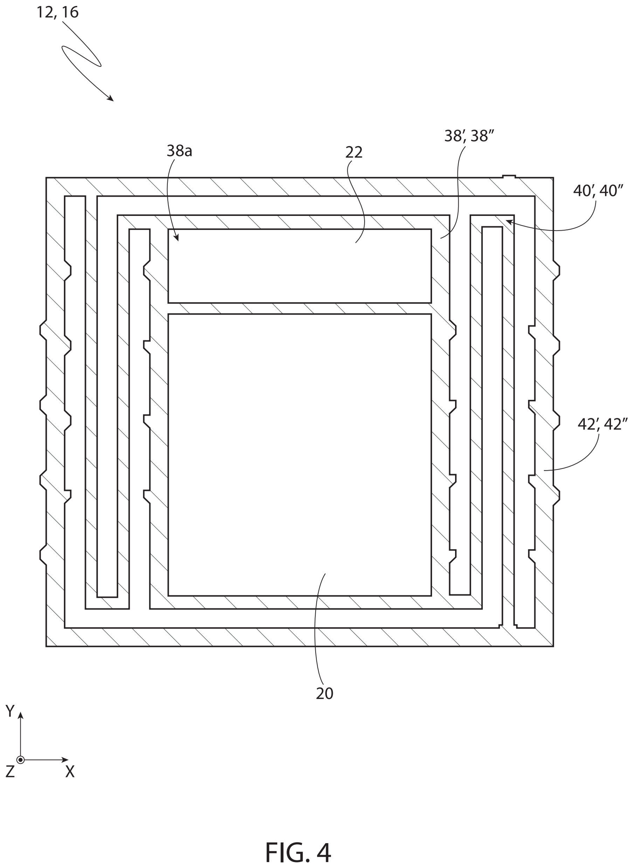

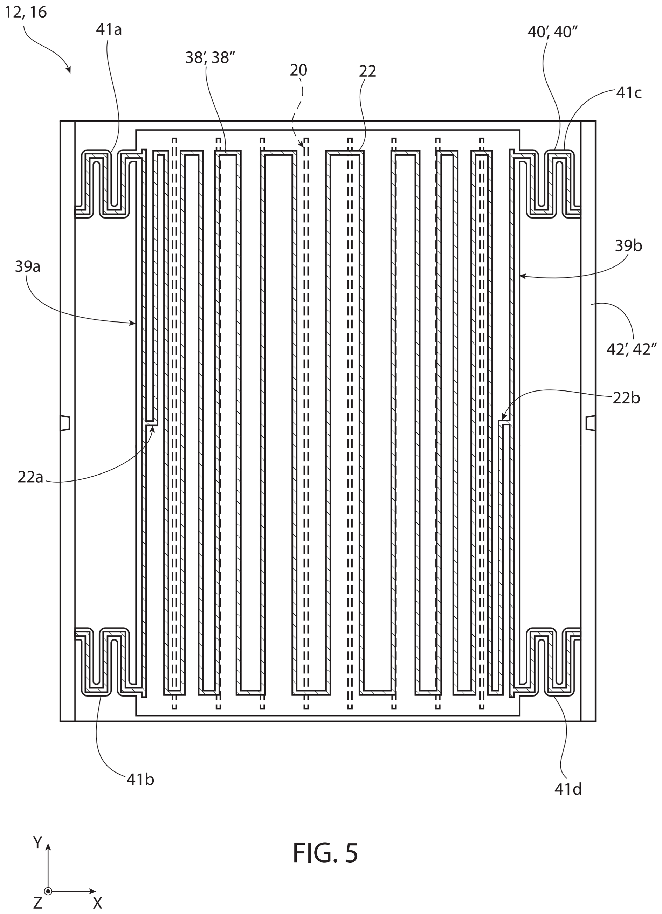

In fact, as better described below with reference to Figures 4 and 5, the detection sensor 12 comprises one or more thermally isolated metal-oxide semiconductor, MOS, TMOS, transistors 20, placed in direct fluidic communication with the air to be analyzed. Hereinafter, the case of a single TMOS 20 is exemplarily considered, nevertheless it becomes apparent that the detection sensor 12 may comprise a plurality of TMOS 20 arranged side by side to each other, for example in such a way as to define a matrix of TMOS 20.

The detection sensor 12 also comprises a heater element 22, such as a resistor of conductive material, configured to emit infrared (IR) radiation when electrically biased, for example by Joule effect.

Together, the TMOS 20 and the heater element 22 form an active group of the detection sensor 12.

The TMOS is a field effect transistor typically used in sensor applications to measure local temperature variations caused for example by absorption of IR radiation. In this case, the heater element 22 generates a predefined and known amount of heat (as it results from the electrical control used for the heater element 22) which tends to heat the detection sensor 12 to a target temperature. However, part of the heat generated by the heater element 22 is dissipated by the air around the detection sensor 12, causing a temperature variation of the detection sensor 12 with respect to the target temperature. This temperature variation, which depends on the thermal conductivity of the air (e.g., a higher thermal conductivity of the air determines a higher thermal absorption by the air, therefore a higher thermal dissipation and a higher temperature variation), is here measurable through the TMOS 20. In fact, this temperature variation causes the generation of charge carriers at the conductive channel of the TMOS 20 and, therefore, a corresponding variation of an output current of the TMOS 20; the latter may then be related to the measured temperature variation, in such a way as to have a measure of the amount of heat which depends on the dissipation in the air, which potentially comprises the target gas. Since the thermal conductivity of the air is influenced in a known manner by the concentration of the target gas in the air, here for example hydrogen, the concentration of the target gas starting from the output current of the TMOS 20 may be measured.

With reference again to Figure 1, the control unit 14 is an electronic unit (such as a microprocessor, a dedicated processor, a CPU, an FPGA, etc.) coupled, for example electrically coupled, to the detection sensor 12.

The control unit 14 is configured to receive the detection signal from the detection sensor 12 and, as a function of the detection signal and as better described below, determine the concentration of the target gas in the air.

For this purpose, the control unit 14 may comprise, in a manner not shown or described in detail as it is known per se, analog-to-digital converters, filtering stages, amplification stages, comparison stages, data storage modules (e.g., memories, such as non-volatile memories), etc.

According to one embodiment, the measuring device 10 further comprises a reference sensor (or second sensor) 16, also operationally coupled to the control unit 14 and configured to generate a reference signal.

The structure and operation of the reference sensor 16 are entirely analogous to those of the detection sensor 12, except for the fact that the one or more TMOS 20 of the reference sensor 16 are fluidically insulated from the air to be analyzed and are therefore not influenced by the concentration of the target gas in the surrounding environment.

This implies that the reference signal generated by the reference sensor 16 is independent of the contribution of the target gas: instead, it is correlated both to the amount of heat generated by the heater element 22 of the reference sensor 16 and to a "baseline" of heat absorption by the air in the absence of the gas to be detected, which determines a standard heat dissipation due to the thermal conductivity of the other gases normally present in the air. Consequently, the reference signal may be used as a reference parameter of the thermal dissipation in air in the absence of the target gas, to take into account secondary effects such as geometry and design of the TMOS 20 and dissipative contributions of the other gases present in the air. In general, this improves the measurement accuracy of the concentration of the target gas.

In this case, therefore, the control unit 14 is configured to receive both the detection signal from the detection sensor 12 and the reference signal from the reference sensor 16 and, as a function of both the detection signal and the reference signal and as better described below, determine the concentration of the target gas in the air.

As schematically shown in Figure 1, the detection sensor 12 and the reference sensor 16 may be integrated in a same micro-electromechanical (MEMS) structure so as to form an assembly (also referred to as measuring assembly or more generically sensor) 30 of the measuring device 10.

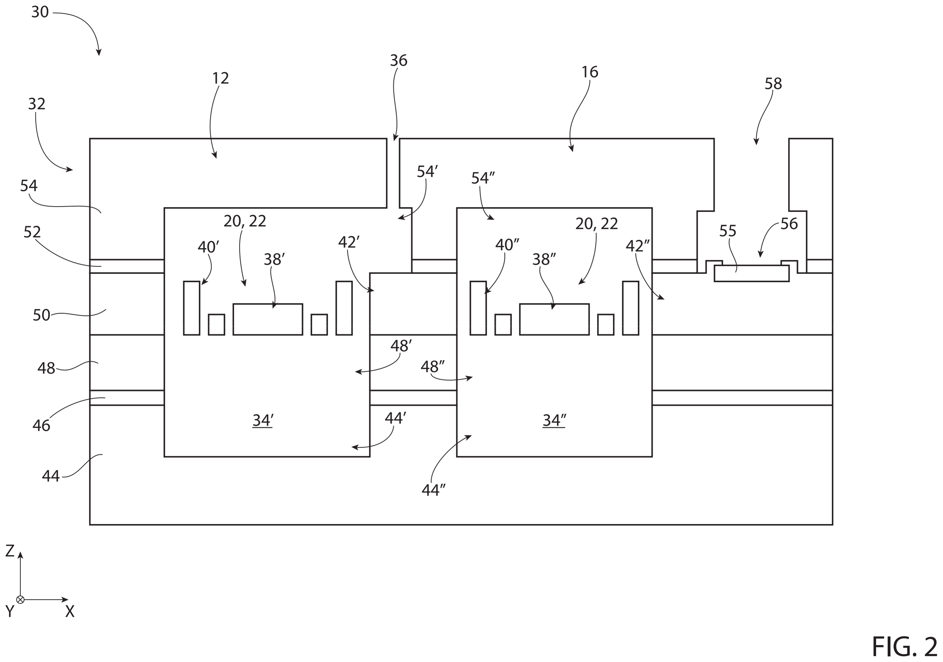

Figure 2 shows a cross-section of this assembly 30.

As seen in Figure 2, the detection sensor 12 and the reference sensor 16 are lateral to each other in the assembly 30, that is, they are arranged side by side, for example, along the X-axis.

In detail, the assembly 30 has a main body 32 which accommodates both sensors 12 and 16.

The main body 32 has a first cavity (or first chamber) 34' and a second cavity (or second chamber) 34", both buried in the main body 32 and lateral to each other, for example along the X-axis.

The first cavity 34' is in fluidic communication with the environment external to the assembly 30 through a communication opening 36 provided in the main body 32, while the second cavity 34" is fluidically insulated with respect to the external environment. The first cavity 34' and the second cavity 34" are therefore not in fluidic connection, i.e. they are mutually insulated.

In detail, the air in the first cavity 34' is the same, in terms of composition and pressure, as the air outside the assembly 30. The air in the second cavity 34" instead may be analogous to the air outside the assembly 30, except for the presence of the target gas, or it may be of a controlled type in terms of pressure and composition.

Hereinafter, it is exemplarily considered the case in which the air in the second cavity 34" has a standard composition, in which the target gas is absent (e.g., it is pure nitrogen or mixtures of nitrogen and other gases such as argon, to simulate real air), and a standard pressure (in particular, it is at a pressure comprised between about 0.5 atm and about 1.5 atm, for example, it is at a pressure equal to about 1 atm). In the present case, with air in which the target gas is absent, reference is made to air which may have in percentage a threshold amount of target gas equal to about 0.01% as a maximum. In fact, the value of 0.01% corresponds to the percentage of secondary gases, including hydrogen, normally present in the air.

A first suspended mass 38' is suspended in the first cavity 34' through a first elastic group 40' which elastically couples the first suspended mass 38' to the main body 32 (in particular, to a first frame portion 42' of the main body 32). Similarly, the second suspended mass 38" is suspended in the second cavity 34" through a second elastic group 40" which elastically couples the second suspended mass 38" to the main body 32 (in particular, to a second frame portion 42" of the main body 32).

The elastic groups 40' and 40" may comprise springs, in particular folded springs such as planar serpentine springs.

The first and the second suspended masses 38', 38" are therefore membranes elastically suspended on the first and the second cavities 34', 34", respectively.

The first suspended mass 38' defines the TMOS 20 and the heater element 22 of the detection sensor 12, while the second suspended mass 38" defines the TMOS 20 and the heater element 22 of the reference sensor 16. In other words, the active group of the detection sensor 12 is accommodated in the first cavity 34' and the active group of the reference sensor 16 is accommodated in the second cavity 34".

The specific structure of each of the sensors 12 and 16 is analogous to known TMOS structures, for example to the structure disclosed in the document EP4428508A1 of the same Applicant.

In particular, the main body 32 may be formed by the following elements, superimposed on each other along the Z-axis: a lower cap 44, a first bonding layer 46, a working substrate 48, a semiconductor body 50, a second bonding layer 52, an upper cap 54.

The working substrate 48 has a first working cavity 48' and a second working cavity 48", lateral to each other along the X-axis and traversing the working substrate 48 along the Z-axis.

The lower cap 44 is fixed, for example by bonding through the first bonding layer 46, to a lower surface of the working substrate 48.

Optionally, the lower cap 44 has a respective first working cavity 44' and a second working cavity 44", lateral to each other along the X-axis and which extend along the Z-axis in the lower cap 44, starting from an upper surface of the lower cap 44 and without reaching a lower surface of the lower cap 44. The working cavities 44', 44" are aligned along the Z-axis respectively with the working cavities 48', 48" and are in direct fluidic communication with the latter through respective through openings of the first bonding layer 46.

The semiconductor body 50 extends on the upper surface of the working substrate 48 and defines the frame portions 42' and 42", the suspended masses 38' and 38" and the elastic groups 40' and 40". In detail, the parts of the semiconductor body 50 which are superimposed along the Z-axis on the working substrate 48 define the frame portions 42' and 42", while the parts of the semiconductor body 50 which are suspended over the working cavities 48' and 48" define the suspended masses 38' and 38" and the elastic groups 40' and 40".

In greater detail and in a manner not shown in Figure 2, the frame portions 42' and 42", the spring groups 40' and 40" and the suspended masses 38' and 38" may comprise one or more semiconductor layers of semiconductor material (e.g., silicon), one or more insulating layers of insulating material (e.g., of silicon oxide) and one or more conductive layers (e.g., of metal such as aluminum).

The upper cap 54 is fixed, for example by bonding through the second bonding layer 52, to an upper surface of the frame portions 42', 42".

Optionally, the upper cap 54 has a respective first working cavity 54' and a second working cavity 54", lateral to each other along the X-axis and which extend along the Z-axis in the upper cap 54, starting from a lower surface of the upper cap 54 and without reaching an upper surface of the upper cap 54. The working cavities 54', 54" are aligned along the Z-axis respectively with the working cavities 48', 48" and are in direct fluidic communication with the latter through respective through openings of the second bonding layer 52 and through the empty regions of the semiconductor body 50 at the suspended masses 38' and 38" and the elastic groups 40' and 40".

The first working cavity 48', the empty regions of the semiconductor body 50 at the first suspended mass 38' and the first elastic group 40' and, if present, the first working cavities 54' and 44' together define the first cavity 34'. Consequently, the first frame portion 42', the working substrate 48 and part of the caps 44 and 54 together define lateral walls of the first cavity 34', while the remaining parts of the caps 44 and 54 define a lower wall and an upper wall of the first cavity 34', respectively.

Similarly, the second working cavity 48", the empty regions of the semiconductor body 50 at the second suspended mass 38" and the second elastic group 40" and, if present, the second working cavities 54" and 44" together define the second cavity 34". Consequently, the second frame portion 42", the working substrate 48 and part of the caps 44 and 54 together define lateral walls of the second cavity 34", while the remaining parts of the caps 44 and 54 define a lower wall and an upper wall of the second cavity 34", respectively.

Furthermore, the upper cap 54 has the previously mentioned communication opening 36, i.e. a through opening which traverses the upper cap 54 along the Z-axis, starting from an upper surface of the upper cap 54 and down to reaching a lower surface of the upper cap 54. In this manner, the communication opening 36 fluidically connects the first cavity 34' with the external environment.

As shown in Figure 2, the assembly 30 may also comprise one or more contact pads 56, for example a plurality thereof for each sensor 12, 16. In the example of Figure 2 only one contact pad 56 is shown, nevertheless it becomes apparent that the number of contact pads 56 may vary based on the design requirements.

In detail, in Figure 2 the contact pad 56 is lateral to the sensors 12 and 16, i.e. faces them along the X-axis. Nevertheless, it becomes apparent that other arrangements may similarly be considered.

In greater detail, the contact pad 56 is formed by a conductive layer 55 (of conductive material, in particular metal such as aluminum) which extends in a contact cavity 58 provided in the upper cap 54, starting from the upper surface of the upper cap 54 and to exposing a part of the upper surface of the semiconductor body 50, where the contact pad 56 is located. Consequently, the contact pad 56 faces towards the outside of the assembly 30, so that it may be electrically contacted through electrical connections external to the assembly 30, not shown.

The contact pad 56 is in electrical connection with the active groups of the sensors 12, 16, for example through electrical connections, not shown in the Figure, which extend in the semiconductor body 50.

The manufacturing process for forming the sensors 12 and 16 is analogous to the sequence of steps disclosed in the document EP4428508A1 of the same Applicant.

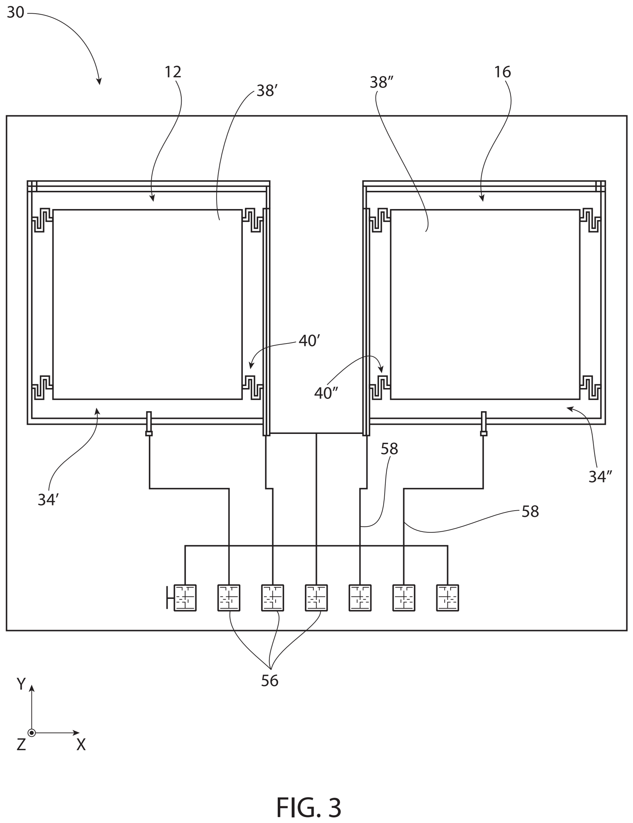

Figure 3 shows a top view with parts removed of the assembly 30, according to an exemplary embodiment.

As exemplarily shown in Figure 3, the assembly 30 has a plurality of contact pads 56, connected to the active groups of the sensors 12, 16 through electrical connections 58. For example, a part of the electrical connections 58 electrically connects the TMOS 20 with a respective part of the contact pads 56, while a remaining part of the electrical connections 58 electrically connects the heater elements 22 with a respective part of the contact pads 56.

Figure 4 instead shows, in a top view with parts removed, the active group and the elastic group of any of the sensors 12 and 16.

In particular, Figure 4 shows an embodiment in which the upper surface (here indicated with the reference 38a) of the suspended mass 38', 38" is defined in part by the TMOS 20 and in part by the heater element 22, which are here exemplarily arranged side by side to each other along the Y-axis. In other words, in this embodiment the conductive regions of the TMOS 20 and the heater element 22 are at the same level along the Z-axis.

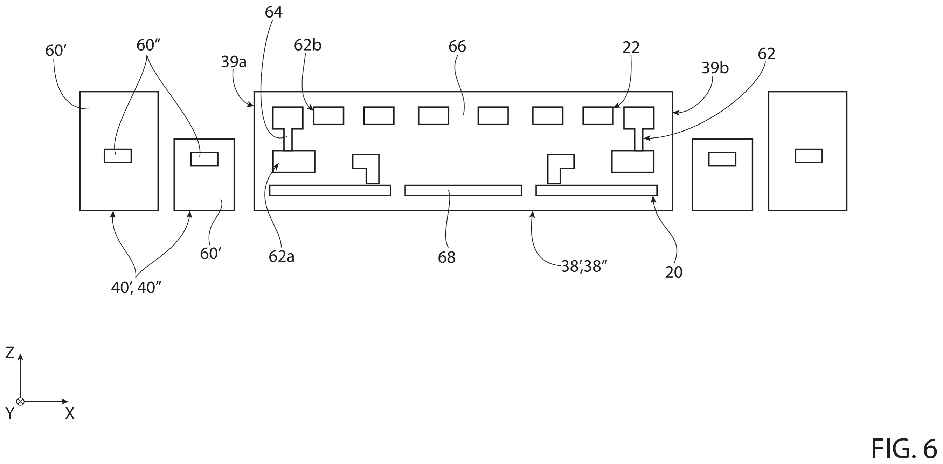

Nevertheless, other embodiments may be similarly considered. For example, the TMOS 20 and the heater element 22 may be arranged on different levels along the Z-axis, as discussed below with reference to Figures 5 and 6.

In the embodiment of Figure 4, the elastic group 40', 40" comprises, exemplarily, two springs, in particular of the serpentine type, facing the sides of the suspended mass 38', 38" which are opposite to each other along the X-axis. For example, the springs have first ends fixed to the respective frame portion 42', 42" (here exemplarily to surfaces of the respective frame portion 42', 42" which are opposite to each other along the Y-axis) and second ends fixed to vertices opposite to each other of the suspended mass 38', 38".

Nevertheless, other shapes and arrangements of the elastic groups 40', 40" may similarly be considered, in a manner known per se.

Figure 5 shows, in top view with parts removed, the active group and the elastic group of any of the sensors 12 and 16, according to an embodiment in which the TMOS 20 and the heater element 22 are arranged on different levels along the Z-axis. Furthermore, Figure 6 shows the suspended mass 38', 38" and the elastic group 40', 40" in cross-section, according to this same embodiment.

In the case of Figures 5 and 6, the heater element 22 is superimposed on the TMOS 20 along the Z-axis.

In the example of Figure 5, the heater element 22 is of the elongated type (e.g., it has a strip shape) and defines a serpentine path along the XY plane, in particular having a plurality of turns which are arranged in succession to each other continuously along the X-axis. The heater element 22 has a first end 22a and a second end 22b, opposite to each other along the serpentine path (i.e. along the X-axis) .

In detail, the serpentine path of the heater element 22 may comprise turns which are closer to each other at the ends 22a and 22b and more spaced at a central portion of the heater element 22, which is interposed between the ends 22a and 22b along the X-axis (in detail, it is arranged centrally between the ends 22a and 22b). In other words, in the example of Figure 5 the distance (or pitch) between consecutive turns of the heater element 22 is smaller at the ends 22a and 22b and is greater at the central portion of the heater element 22. This increases the temperature uniformity of the suspended mass 38', 38".

For instance, the distance between turns of the heater element 22 which are consecutive to each other increases continuously starting from the ends 22a and 22b until reaching the central portion of the heater element 22.

The turns of the heater element 22 may instead have a cross-section analogous to each other (e.g., same thickness along the Z-axis and same width in the direction transversal to the turn).

For purely illustrative purposes, the heater element 22 has an electrical resistance comprised between about 100 Ω and about 2 kΩ.

In the embodiment of Figures 5 and 6, the elastic group 40', 40" comprises, exemplarily, four springs, in particular of the serpentine type, facing each other two by two on the sides of the suspended mass 38', 38" which are opposite to each other along the X-axis and arranged at the vertices of the suspended mass 38', 38". In other words, the suspended mass 38', 38", shown here exemplarily with a quadrangular shape in top view (e.g. square shape), has a first side (or first lateral wall) 39a and a second side (or second lateral wall) 39b, opposite to each other along the X-axis and facing the frame portion 42', 42" through the cavity 34', 34"; a first pair of springs 41a and 41b faces the first side 39a, while a second pair of springs 41c and 41d faces the second side 39b. Each of the springs 41a-41d is coupled to the suspended mass 38', 38" at a respective vertex of the latter, in top view. Alternatively, a plurality of springs may also be considered for each side 39a and 39b, for example three or more springs for each side 39a and 39b.

For instance, the springs 41a-41d have first ends fixed to the respective frame portion 42', 42" (here exemplarily to surfaces of the respective frame portions 42', 42" which are opposite to each other along the X-axis) and second ends fixed to the respective vertices of the suspended mass 38', 38".

Nevertheless, other shapes and arrangements of the elastic groups 40', 40" may similarly be considered, in a manner known per se.

As shown in Figure 6, the springs 41a-41d comprise a main body 60' of insulating material (e.g., silicon oxide) and electrical connections (hereinafter also referred to as routing connections) 60" of conductive material (e.g., metal such as aluminum), which are buried in the main body 60' so as to be electrically insulated from the cavity 34', 34".

The routing connections 60" extend within the respective springs 41a-41d along their serpentine path, in such a way as to extend continuously between the first ends and the second ends of the springs 41a-41d and thus create a conductive path through the springs 41a-41d.

The routing connections 60" are electrically connected to the heater element 22 and the TMOS 20 in such a way as to electrically connect these components to the contact pads 56. Although this is not shown in detail in the Figure, it becomes apparent that first routing connections 60" are present between the heater element 22 and the one or more respective contact pads 56, and second routing connections 60" are present between the TMOS 20 and the one or more respective contact pad 56, and that the first and the second routing connections 60" are electrically decoupled from each other. This allows simultaneously the electrical biasing of the heater element 22 and the acquisition of the detection/reference signal from the TMOS 20.

In a manner known per se, the electrical connection between the springs 41a-41d and the contact pads 56 occurs through extensions of the routing connections through the frame portions 42', 42" or through further electrical connections, not shown, which traverse the frame portions 42', 42" and connect the routing connections 60" and the contact pads 56 to each other.

For instance, the first and the second routing connections 60" may be on metallization levels different from each other along the Z-axis, or they may be lateral to each other on the same metallization level. In both cases the first and the second routing connections 60" are electrically insulated from each other through insulating material interposed therebetween.

In particular, the first routing connections 60" of the pair of springs 41a and 41b are parallel-connected to each other at the first end 22a of the heater element 22, and the first routing connections 60" of the pair of springs 41c and 41d are parallel-connected to each other at the second end 22b of the heater element 22. In this manner, a series circuit is created comprising, in series with each other, a first circuit (formed by the first routing connections 60" of the pair of springs 41a and 41b, in parallel with each other), a second circuit (formed by the heater element 22) and a third circuit (formed by the first routing connections 60" of the pair of springs 41c and 41d, in parallel with each other). This minimizes the effect, on the heater element 22, of parasitic series resistances due to the first routing connections 60".

As shown in Figure 6, the suspended mass 38', 38" comprises a main body 66 of insulating material (e.g., of silicon oxide), having one or more semiconductor layers 68 of semiconductor material (e.g., silicon) and one or more metallizations 62 of conductive material (in particular metal such as aluminum) accommodated therein.

In Figure 6, a first metallization 62a on a first metallization level and a second metallization 62b on a second metallization level, superimposed on the first metallization level along the Z-axis, are exemplarily shown; nevertheless, it becomes apparent that a greater number of metallizations 62 may similarly be considered, in particular for forming the TMOS 20. The metallizations 62 are buried in the main body 66 in such a way as not to be in mutual direct electrical contact and are joined through conductive vias which extend through the main body 66 between the different metallization levels.

The second metallization 62b defines the serpentine path of the heater element 22. The first metallization 62a, which for example is at the same level, along the Z-axis, as the routing connections 60" (in detail, at least as the first routing connections 60"), forms connections between the ends 22a and 22b of the heater element 22 and the routing connections 60".

The one or more semiconductor layers 68, which are shown here below the metallizations 62, form part of the TMOS 20. For example, the TMOS 20 is also defined by part of the first metallization 62a (or by one or more other metallizations 62, not shown here and underlying the first metallization 62a) and by a part of the main body 66 placed at the one or more semiconductor layers 68, in a manner known per se and therefore not further described here in detail.

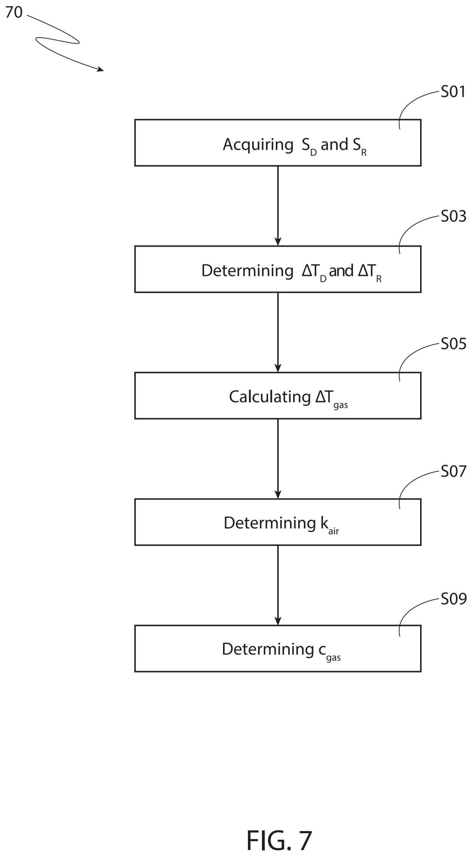

In use, the measuring device 10 implements a measuring method 70, shown in Figure 7 and now described in detail.

The measuring method 70 is described here with reference to the embodiment in which the reference sensor 16 is also present, nevertheless it becomes apparent that it may be generalized to the case in which the reference sensor 16 is absent.

At a step S01, the control unit 14 acquires the detection signal (here indicated with the reference S_{D}) from the detection sensor 12 and the reference signal (here indicated with the reference S_{R}) from the reference sensor 16.

At a step S03, the control unit 14 determines a detection temperature difference ΔT_{D} and a reference temperature difference ΔT_{R}, starting from the detection signal S_{D} and the reference signal S_{R}, respectively.

In particular, the detection temperature difference ΔT_{D} depends on the difference between the temperature value of the detection signal S_{D} (also referred to as measured detection temperature T_{D}) and a reference temperature T_{H}, in particular the temperature of the heater element 22 of the detection sensor 12. In detail, the detection temperature difference ΔT_{D} is equal to this difference (i.e. ΔT_{D}=T_{D}-T_{H}). Similarly, the reference temperature difference ΔT_{R} depends on the difference between the temperature value of the reference signal S_{R} (also referred to as measured reference temperature T_{R}) and the reference temperature T_{H}, in particular the temperature of the heater element 22 of the reference sensor 16. In detail, the reference temperature difference ΔT_{R} is equal to this difference (i.e. ΔT_{R}=T_{R}-T_{H}).

Since the heater elements 22 of the detection sensor 12 and the reference sensor 16 are electrically controlled in the same manner and in a known manner, the reference temperatures T_{H} that they reach in use are equal to each other (i.e. a mutual offset is lower than, for example, 1% of the reference temperature T_{H}) and known (e.g., they are comprised between about 20°C and about 80°C more than the room temperature of the air around the measuring device 10).

Consequently, the reference temperature difference ΔT_{R} is indicative of an offset of the temperature measured by the TMOS 20 of the reference sensor 16 from the temperature of the heater element 22 of the reference sensor 16; this offset is due to several factors, such as design factors (e.g., the thermal dissipation caused by the elastic group 40') and standard thermal dissipation in air (i.e., the thermal dissipation caused by the thermal conductivity of the air in the absence of the target gas, which in particular depends on the temperature considered), but is independent of any concentration of the target gas in the air around the measuring device 10. Instead, the detection temperature difference ΔT_{D} is indicative of the offset of the temperature measured by the TMOS 20 of the detection sensor 12 from the temperature of the heater element 22 of the detection sensor 12, which depends on both the previously mentioned factors (e.g., design factors and standard thermal dissipation in air) and on any concentration of the target gas in the air around the measuring device 10 (i.e., the additional thermal dissipation due to the thermal conductivity of the target gas, if present).

At a step S05, the control unit 14 calculates a gas temperature difference ΔT_{gas} which depends on the difference between the detection temperature difference ΔT_{D} and the reference temperature difference ΔT_{R}. In detail, the gas temperature difference ΔT_{gas} is equal to this difference (i.e. ΔT_{gas}=ΔT_{D}-ΔT_{R}).

Consequently, the gas temperature difference ΔT_{gas} is indicative of an offset of the temperature measured by the TMOS 20 of the detection sensor 12 from the temperature measured by the TMOS 20 of the reference sensor 16, which is due to any concentration of the target gas in the air around the measuring device 10 (i.e. this additional and possible contribution of thermal dissipation due to the thermal conductivity of the target gas, if present). In other words, the gas temperature difference ΔT_{gas} is zero in the absence of the target gas in the air and instead is non-zero in case of presence of the target gas in the air (in particular, it increases in absolute value as the concentration of the target gas in air increases).

At a step S07, the control unit 14 determines the thermal conductivity kₐᵢᵣ of the air based on the gas temperature difference ΔT_{gas}.

In particular, this is possible because the correlation between the thermal conductivity kₐᵢᵣ of the air and the gas temperature difference ΔT_{gas} is known. For example, this correlation may be determined through heuristic methods, according to techniques that are known per se.

For instance, a look-up table may be present in the control unit 14 which stores the associations between each measured value of gas temperature difference ΔT_{gas} and the respective measured value of thermal conductivity kₐᵢᵣ of the air, or a memory space which stores a mathematical correlation function between thermal conductivity kₐᵢᵣ of the air and the gas temperature difference ΔT_{gas} (e.g., obtained through interpolation of the value pairs of gas temperature difference ΔT_{gas} and thermal conductivity kₐᵢᵣ of the air previously mentioned).

At a step S09, the control unit 14 determines a concentration c_{gas} of the target gas in the air based on the determined thermal conductivity kₐᵢᵣ of the air.

In particular, this is possible because the correlation between the concentration c_{gas} of the target gas in the air and the thermal conductivity kₐᵢᵣ of the air is known. For example, this correlation may be determined through heuristic methods, according to techniques that are known per se.

For instance, a further look-up table may be present in the control unit 14 which stores the associations between each measured value of thermal conductivity kₐᵢᵣ and the respective measured value of concentration c_{gas} of the target gas, or further memory space which stores a further mathematical correlation function between the thermal conductivity kₐᵢᵣ and the concentration c_{gas} of the target gas (e.g., obtained through interpolation of the value pairs of thermal conductivity kₐᵢᵣ and concentration c_{gas} of the target gas previously mentioned).

For instance, the control unit 14 may also determine the presence or absence of the target gas in air, for example by comparing the measured concentration c_{gas} of the target gas with a threshold concentration (e.g., for hydrogen, equal to about 0.01% or about 4%, corresponding to the flammability threshold of hydrogen, or about 10%); in case the measured concentration c_{gas} is greater than the threshold concentration, the presence of the target gas is confirmed, conversely the absence of the target gas is confirmed.

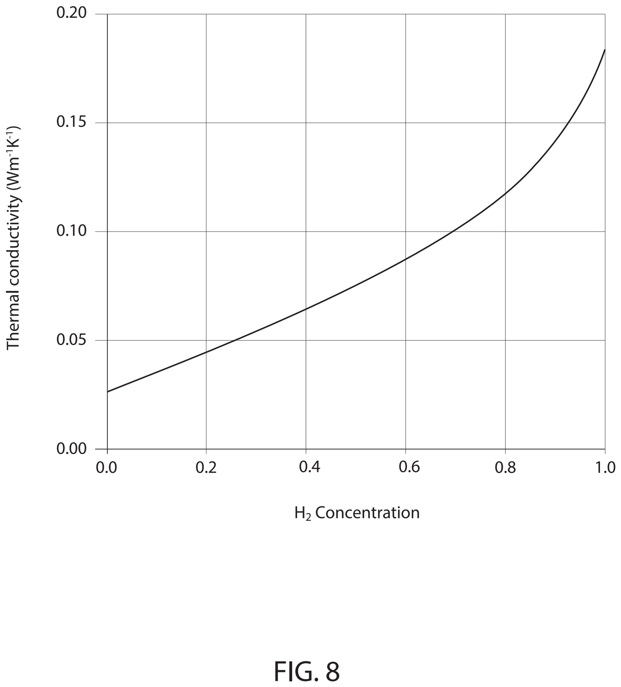

Figure 8 shows an example of a curve which correlates the thermal conductivity kₐᵢᵣ of the air and the concentration c_{gas} of the target gas in air, in particular as regards hydrogen.

As seen in Figure 8, a concentration c_{gas} of hydrogen in air of about zero causes a thermal conductivity kₐᵢᵣ of the air equal to about 0.02 W/(m·K), a concentration c_{gas} of hydrogen in air equal to about 100% causes a thermal conductivity kₐᵢᵣ of the air equal to about 0.18 W/(m·K), and a concentration c_{gas} of hydrogen in air equal to about 50% causes a thermal conductivity kₐᵢᵣ of the air equal to about 0.08 W/(m·K).

As previously mentioned, the measuring method 70 is similarly applicable to the case in which the reference sensor 16 is absent.

In this case, at step S01 only the detection signal S_{D} is acquired, at step S03 only the detection temperature difference ΔT_{D} is determined, step S05 is absent, at step S07 the thermal conductivity kₐᵢᵣ of the air is determined based on the detection temperature difference ΔT_{D} (in an entirely analogous manner to what has been previously described), and finally at step S09 the concentration c_{gas} of the target gas in the air is determined based on the determined thermal conductivity kₐᵢᵣ of the air.

The measuring method 70 is implementable in the control unit 14 through a corresponding computer program product, which is not described in detail again as it is evident in view of the preceding discussion of the measuring method 70.

From an examination of the characteristics of the invention made according to the present invention, the advantages that it affords are evident.

In particular, the measuring device 10 allows an accurate measurement of the concentration of target gas in air.

This is made possible by virtue of the differential reading which is performed also considering the reference measurement of the reference sensor 16.

For instance, the differential reading allows to compensate for the variations in the standard conductivity of the air as a function of the temperature, unlike the currently known solutions.

Furthermore, the use of the TMOS 20 allows this result to be achieved with a reduced power consumption, smaller dimensions of the assembly 30 and a lower manufacturing cost of the measuring device 10.

Finally, it is clear that modifications and variations may be made to the invention described and illustrated here without thereby departing from the scope of the present invention, as defined in the attached claims.

For instance, the different embodiments described may be combined with each other to provide further solutions.

Furthermore, even target gases other than hydrogen may be detected in an analogous manner to what has been described so far (e.g., carbon dioxide, helium, methane, etc.). In this case, it is sufficient to consider a different association between the concentration c_{gas} of the target gas in the air and the thermal conductivity kₐᵢᵣ of the air.

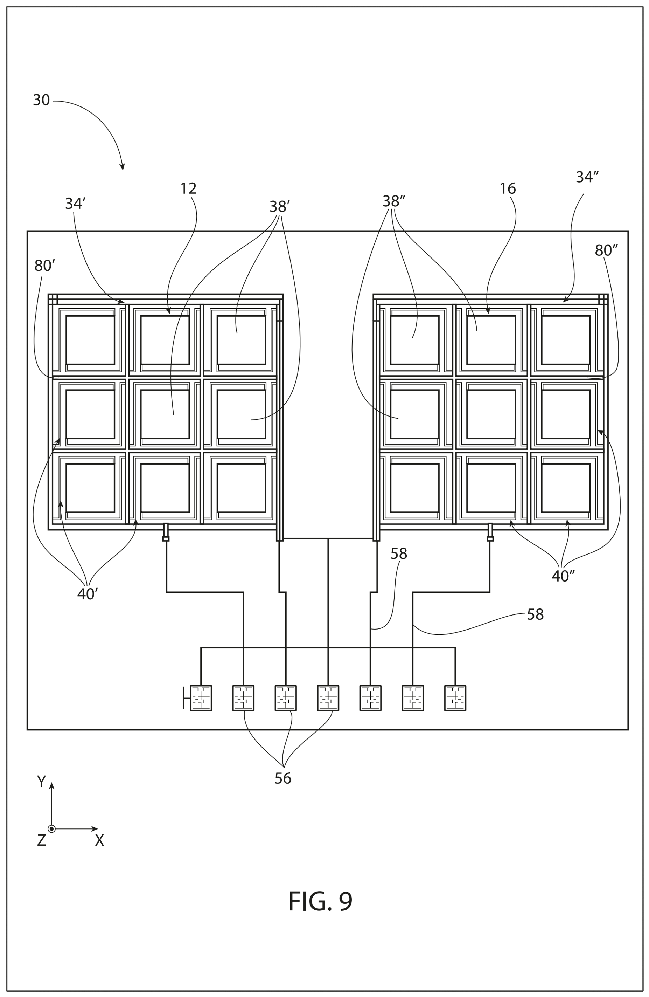

Furthermore, although the case in which each sensor 12, 16 comprises a single suspended mass 38', 38" has been exemplarily discussed so far, it is also possible to have the sensors 12 and 16 each comprising a respective plurality of suspended masses 38', 38". Figure 9 shows an example of this case.

In the example of Figure 9, each cavity 34', 34" accommodates a respective plurality of suspended masses 38', 38", suspended therewithin. In each of the cavities 34', 34", the suspended masses 38', 38" are arranged side by side and aligned with each other, for example at the same level along the Z-axis, so as to define an array or a matrix of suspended masses 38', 38" (Figure 9 exemplarily shows a matrix, in top view).

In this case, the suspended masses 38', 38" of each sensor 12, 16 may be electrically connected in parallel to each other.

The suspended masses 38', 38" of each sensor 12, 16 are elastically coupled to each other and/or with respect to the respective frame portion 42', 42", for example through the respective elastic groups 40', 40".

In detail, for illustrative and non-limiting purposes, each of the sensors 12 and 16 also comprises a respective suspended frame portion 80', 80", fixed to the respective frame portion 42', 42" and suspended in the respective cavity 34', 34", as shown in Figure 9. The suspended frame portion 80', 80" has a grid shape and defines a frame structure suspended in the respective cavity 34', 34" and integral with the respective suspended frame portion 80', 80". The suspended masses 38', 38" are elastically coupled, through the respective elastic groups 40', 40", to the respective suspended frame portion 80', 80" and consequently to the respective frame portion 42', 42". For example, the suspended masses 38', 38" each extend within a respective opening of the suspended frame portion 80', 80", level with the latter along the Z-axis.

Nevertheless, other structures and arrangements may similarly be considered to elastically suspend the plurality of suspended masses 38', 38" in the cavities 34', 34".

## Claims

1. A sensor (30), of the micro-electromechanical type, for detecting a target gas in air,
the sensor (30) comprising:
- a main body (32) defining a first cavity (34'), buried in the main body (32) and in fluidic communication with an environment external to the sensor (30) in such a way as to allow the flow of air from the external environment towards the first cavity (34'), the main body (32) comprising a first frame portion (42') facing the first cavity (34');
- a first suspended mass (38'), suspended in the first cavity (34') and comprising at least one thermally isolated metal-oxide semiconductor, TMOS transistor, (20), configured to detect the target gas in air, and at least one heater element (22), configured to heat the first suspended mass (38'); and
- a first elastic group (40') extending in the first cavity (34') between the first suspended mass (38') and the first frame portion (42'), the first elastic group (40') being configured to elastically couple the first suspended mass (38') to the first frame portion (42').

2. The sensor according to claim 1, wherein the main body (32) further defines a second cavity (34") which is buried in the main body (32), is lateral to the first cavity (34') and is fluidically insulated from the environment external to the sensor (30) in such a way as to prevent the flow of air from the external environment towards the second cavity (34"), the main body (32) further comprising a second frame portion (42") facing the second cavity (34"),
the sensor (30) further comprising:
- a second suspended mass (38"), suspended in the second cavity (34") and comprising at least one respective TMOS (20) and at least one respective heater element (22), configured to heat the second suspended mass (38"); and
- a second elastic group (40") extending in the second cavity (34") between the second suspended mass (38") and the second frame portion (42"), the second elastic group (40") being configured to elastically couple the second suspended mass (38") to the second frame portion (42").

3. The sensor according to claim 2, wherein the second cavity (34") is configured to be filled with air, wherein the target gas is absent and at a predefined pressure comprised between 0.5 atm and 1.5 atm.

4. The sensor according to claim 2 or 3, wherein the first suspended mass (38') defines a detection sensor (12) which, through the respective TMOS (20), is configured to generate a detection signal (S_{D}) which depends on a concentration of the target gas in air, and
wherein the second suspended mass (38") defines a reference sensor (16) which, through the respective TMOS (20), is configured to generate a reference signal (S_{R}) which is independent of the concentration of the target gas in air.

5. The sensor according to any of the preceding claims, wherein the TMOS (20) and the heater element (22) are superimposed on each other in the first suspended mass (38').

6. The sensor according to claim 5, wherein the heater element (22) defines a serpentine path along a plane (XY) parallel to an upper surface of the first suspended mass (38'), the serpentine path comprising a plurality of turns, consecutive to each other along a first axis (X) of said plane (XY), and having a first end (22a) and a second end (22b) opposite to each other along the first axis (X),
wherein a distance along the first axis (X) between consecutive turns of the heater element (22) is smaller at the first (22a) and the second (22b) ends and is greater at a central portion of the heater element (22), which is interposed between the first (22a) and the second (22b) ends along the first axis (X).

7. The sensor according to claim 6, wherein the first suspended mass (38') has a first side (39a) and a second side (39b), opposite to each other along the first axis (X) and facing respective sides of the first frame portion (42') through the first cavity (34'),
wherein the first elastic group (40') comprises a first plurality of springs (41a, 41b), extending between the first frame portion (42') and the first side (39a), and a second plurality of springs (41c, 41d), extending between the first frame portion (42') and the second side (39b),
wherein each spring (41a-41d) has a respective first end fixed to the first frame portion (42') and a respective second end fixed to the first suspended mass (38'),
wherein each spring (41a-41d) comprises at least one respective routing connection (60") extending between the first and the second ends of the spring (41a-41d),
wherein the routing connections (60") of the first plurality of springs (41a, 41b) are electrically coupled, in parallel with each other, to the first end (22a) of the heater element (22) and the routing connections (60") of the second plurality of springs (41c, 41d) are electrically coupled, in parallel with each other, to the second end (22b) of the heater element (22).

8. The sensor according to claim 7, wherein the first suspended mass (38') has four vertices in the plane (XY),
wherein the first plurality of springs (41a, 41b) comprises two respective springs and the second plurality of springs (41c, 41d) comprises two respective springs, and
wherein the second end of each spring (41a-41d) is coupled to the first suspended mass (38') at a respective vertex of said four vertices.

9. The sensor according to claim 7 or 8, wherein the first suspended mass (38') comprises a main mass body (66) of insulating material which accommodates one or more semiconductor layers (68) of semiconductor material and at least a first (62a) and a second (62b) metallization of conductive material, the first (62a) and the second (62b) metallizations being superimposed on the one or more semiconductor layers (68) orthogonally to the plane (XY) and the second metallization (62b) being superimposed on the first metallization (62a) orthogonally to the plane (XY),
wherein the second metallization (62b) defines the heater element (22) with a serpentine path,
wherein the first metallization (62a) defines electrical connections between the routing connections (60") and the first (22a) and the second (22b) ends of the heater element (22), and
wherein the one or more semiconductor layers (68) form part of the TMOS (20).

10. The sensor according to any of the preceding claims, the sensor (30) comprising a plurality of said first suspended masses (38'), suspended in the first cavity (34') and each comprising at least one respective TMOS (20) and at least one respective heater element (22), the first suspended masses (38') being elastically coupled to the first frame portion (42') and having an array or a matrix arrangement between each other.

11. A measuring device (10) for detecting a target gas in air,
the measuring device (10) comprising:
- a sensor (30) according to any of the preceding claims, wherein the first suspended mass (38') defines a detection sensor (12) which, through the respective TMOS (20), is configured to generate a detection signal (S_{D}) which depends on a concentration of the target gas in air; and
- a control unit (14), operationally coupled to the detection sensor (12) and configured to receive the detection signal (S_{D}) and, based on the detection signal (S_{D}), determine a concentration of the target gas in air.

12. The measuring device according to claim 11, wherein the main body (32) further defines a second cavity (34") which is buried in the main body (32), is lateral to the first cavity (34') and is fluidically insulated from the environment external to the measuring device (10) in such a way as to prevent the flow of air from the external environment towards the second cavity (34"), the main body (32) further comprising a second frame portion (42") facing the second cavity (34"),
the sensor (30) further comprising:
- a second suspended mass (38"), suspended in the second cavity (34") and comprising at least one respective TMOS (20) and at least one respective heater element (22), configured to heat the second suspended mass (38"); and
- a second elastic group (40") extending in the second cavity (34") between the second suspended mass (38") and the second frame portion (42"), the second elastic group (40") being configured to elastically couple the second suspended mass (38") to the second frame portion (42"),
wherein the second suspended mass (38") defines a reference sensor (16) which, through the respective TMOS (20), is configured to generate a reference signal (S_{R}) which is independent of the concentration of the target gas in air, and
wherein the control unit (14) is also operationally coupled to the reference sensor (16) and is further configured to also receive the reference signal (S_{R}) and, also based on the reference signal (S_{R}), determine the concentration of the target gas in air.

13. A measuring method (70) for detecting a target gas in air,
the measuring method (70) being performed by a measuring device (10) according to claim 11 or 12,
the measuring method (70) comprising the steps of:
acquiring (S01), by the control unit (14) and through the detection sensor (12), the detection signal (S_{D});
determining (S03), by the control unit (14) and based on the detection signal (S_{D}), a detection temperature difference (ΔT_{D}) indicative of an offset of a temperature measured by the TMOS (20) of the detection sensor (12) with respect to a temperature of the heater element (22) of the detection sensor (12), said offset depending on a thermal dissipation of the air in the presence of the target gas;
determining (S07), by the control unit (14), a thermal conductivity (kₐᵢᵣ) of the air based on the detection temperature difference (ΔT_{D}); and
determining (S09), by the control unit (14), a concentration (c_{gas}) of the target gas in the air based on the thermal conductivity (kₐᵢᵣ) of the air.

14. The measuring method according to claim 13, wherein the main body (32) further defines a second cavity (34") which is buried in the main body (32), is lateral to the first cavity (34') and is fluidically insulated from the environment external to the measuring device (10) in such a way as to prevent the flow of air from the external environment towards the second cavity (34"), the main body (32) further comprising a second frame portion (42") facing the second cavity (34"),
the sensor (30) further comprising:
- a second suspended mass (38"), suspended in the second cavity (34") and comprising at least one respective TMOS (20) and at least one respective heater element (22), configured to heat the second suspended mass (38"); and
- a second elastic group (40") extending in the second cavity (34") between the second suspended mass (38") and the second frame portion (42"), the second elastic group (40") being configured to elastically couple the second suspended mass (38") to the second frame portion (42"),
wherein the second suspended mass (38") defines a reference sensor (16) which, through the respective TMOS (20), is configured to generate a reference signal (S_{R}) which is independent of the concentration of the target gas in air, and
wherein the control unit (14) is also operationally coupled to the reference sensor (16) and is further configured to also receive the reference signal (S_{R}) and, also based on the reference signal (S_{R}), determine the concentration of the target gas in air,
wherein the step of acquiring (S01) the detection signal (S_{D}) further comprises acquiring, by the control unit (14) and through the reference sensor (16), the reference signal (S_{R}),
wherein the step of determining (S03) the detection temperature difference (ΔT_{D}) further comprises determining, by the control unit (14) and based on the reference signal (S_{R}), a reference temperature difference (ΔT_{R}) indicative of an offset of a temperature measured by the TMOS (20) of the reference sensor (16) with respect to a temperature of the heater element (22) of the reference sensor (16), said offset depending on a thermal dissipation of the air in the absence of the target gas,
wherein the measuring method (70) further comprises calculating (S05), by the control unit (14), a gas temperature difference (ΔT_{gas}) dependent on a difference between the detection temperature difference (ΔT_{D}) and the reference temperature difference (ΔT_{R}), and
wherein the step of determining (S07) the thermal conductivity (kₐᵢᵣ) of the air comprises determining, by the control unit (14), the thermal conductivity (kₐᵢᵣ) of the air based on the gas temperature difference (ΔT_{gas}), also dependent on the reference temperature difference (ΔT_{R}).

15. A computer program product storable in a measuring device (10) according to claim 11 or 12,
the computer program being designed such that, when executed, the measuring device (10) becomes configured to perform a measuring method (70) according to claim 13 or 14.
